# EUROPÄISCHE PATENTANMELDUNG

(11) **EP 1 288 197 A2**
(43) Veröffentlichungstag der Anmeldung: **05.03.2003**
(21) Anmeldenummer: 02018160.8
(22) Anmeldetag: 19.08.2002
(51) Int. Cl.: C07C 305/04, C07C 303/24

(54) **N-unsubstituierte Amidinium-Salze**

(30) Priorität: 30.08.2001 DE 10142335
(71) Anmelder: Bayer Aktiengesellschaft, 51368 Leverkusen (DE)
(72) Erfinder: Kraus, Helmut, Dr., 51519 Odenthal (DE); Wodarsch, Rosemarie, 51469 Bergisch Gladbach (DE)

(57) **Zusammenfassung**

Die vorliegende Erfindung betrifft N-unsubstituierte Imidsäureester-Alkylsulfate, N-unsubstituierte Amidinium-Alkylsulfate und ein Verfahren zu deren Herstellung das dadurch gekennzeichnet ist, dass Carbonsäureamide mit Schwefelsäurediestern in die entsprechenden Imidsäureester-Alkylsulfate überführt werden, und diese weiterhin mit Ammoniak zu den analogen Amidinium-Alkylsulfaten umgesetzt werden.

## Beschreibung

Die Erfindung betrifft N-unsubstituierte Amidinium-Salze, deren Vorprodukte und ein Verfahren zu ihrer Herstellung.

N-unsubstituierte Amidine und deren Salze, im Folgenden vereinfacht Amidine und Amidinium-Salze genannt, sind wertvolle Synthesebausteine bei der Herstellung von heterocyclischen Verbindungen. Solche Verbindungen finden vielfältige Anwendung insbesondere im Pharmabereich und im Pflanzenschutz. Beispielsweise können Vitamin B1, Eprosartan und Conivaptan vorteilhaft aus Alkylamidinen oder Alkylamidinium-Salzen hergestellt werden.

Alkylamidinium-Salze, wie beispielsweise Acetamidinium-chlorid, werden technisch nach der sogenannten Pinner-Synthese hergestellt (Organic Synthesis Col. Vol I, S: 4; RO 59061; Izobreteniya 1995(32), 264). Dabei wird zunächst aus Acetonitril, Methanol und HCl das entsprechende Imidsäureester-Hydrochlorid synthetisiert.

In der Literatur findet man für den Begriff Imidsäureester auch häufig die Bezeichnungen Imidoester, Iminoester, Imidoether, Imidat oder Iminoether, die jedoch äquivalent sind.

In einem zweiten Schritt wird das schwerlösliche und daher zwischenisolierte Imidsäureester-Hydrochlorid mit einer Dosierschnecke zu einer Ammoniaklösung dosiert. Dabei fällt das ebenfalls schwerlösliche Acetamidin-Hydrochlorid an. Das beschriebene Verfahren hat den Nachteil, dass wegen der starken Hygroskopizität und Thermolabilität des Zwischenprodukts sowie dessen Einsatz als Feststoff besondere prozesstechnische Vorkehrungen getroffen werden müssen, um Verluste zu vermeiden. Aus den gleichen Gründen ist auch die Handhabung des festen Acetamidin-Hydrochlorids unvorteilhaft.

Diese Nachteile können laut JP-A-60-84254 umgangen werden, wenn Acetonitril und Chlorwasserstoff in einem bestimmten Verhältnis miteinander umgesetzt werden Bei Einsatz von 1,35 bis 2 Äquivalenten Chlorwasserstoff wird eine Aufschlämmung, bei mehr als 2 Äquivalenten eine Lösung erhalten. Diese überschüssige Säure muss jedoch vor der Umsetzung mit Ammoniak sehr vorsichtig neutralisiert werden, um Überhitzung und die bereits erwähnte thermische Zersetzung zu vermeiden. Diese Maßnahme bedeutet einen zusätzlichen Reaktionsschritt und die Bildung von unerwünschten Salzmengen und ist daher für die technische Realisierung unvorteilhaft.

Es bestand daher das Bedürfnis, ein technisch gangbares Verfahren zur Herstellung von Amidinen und Amidinium-Salzen zu entwickeln, das zusätzliche Neutralisationsschritte oder die prozesstechnisch aufwändige Zwischenisolierung schwerlöslicher Imidsäureester-Salze weitestgehend vermeidet.

Es wurde nun ein Verfahren zur Herstellung von Amidinium-Salzen gefunden, das dadurch gekennzeichnet ist, dass Carbonsäureamide der allgemeinen Formel (I) 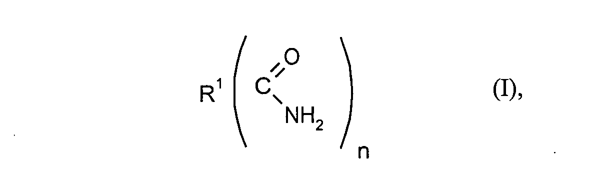 in der
- n: für eins oder zwei und abhängig von n
für n=1
- R¹: für C₁-C₁₂-Alkyl, C₂-C₈-Alkenyl, C₁-C₁₂-Halogenalkyl oder C₇-C₁₂-Arylalkyl oder Substituenten der allgemeinen Formel (II)

A-B-D (II)
in der unabhängig voneinander
- A: für einen C₁-C₄-Alkylenrest und
- B: für eine Carbonylgruppe und
- D: für R² oder OR² steht,
wobei R² C₁-C₆-Alkyl bedeutet oder
B und D zusammen für eine Cyanogruppe stehen,
und für n=2
- R¹: für C₁-C₆-Alkylen oder C₂-C₆-Alkenylen steht,

a) gegebenenfalls in Gegenwart von Lösungsmittel mit mindestens einem Schwefelsäurediester der allgemeinen Formel (III) 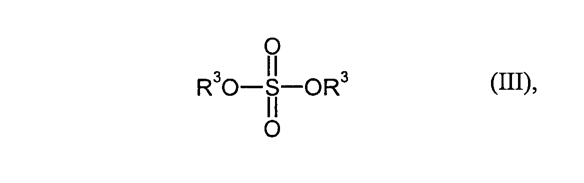 in der die Reste R³ jeweils unabhängig voneinander für C₁-C₆-Alkyl stehen, zu Imidsäureester-Alkylsulfaten der allgemeinen Formel (IV) 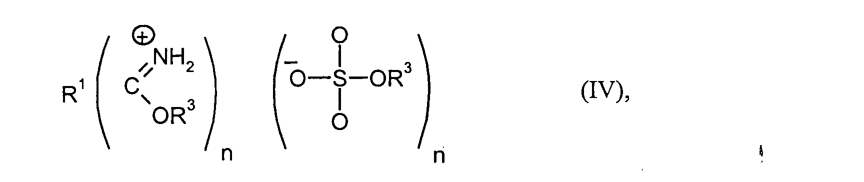 in der
   n, R¹ und jeweils unabhängig voneinander die Reste R³ die oben genannte Bedeutung besitzen., umgesetzt werden und
b) diese dann gegebenenfalls in Gegenwart von Lösungsmittel mit Ammoniak zu den Amidinium-Alkylsulfaten der allgemeinen Formel (V) umgesetzt werden, 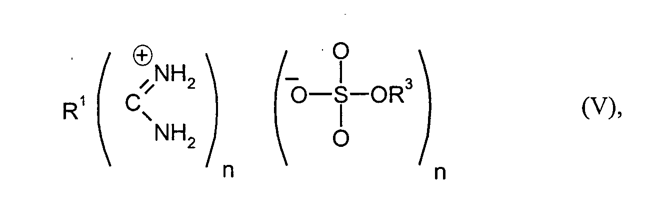 in der
   n, R¹ und R³ die oben genannte Bedeutung besitzen.

### Weiterhin können Amidine der allgemeinen Formel (VI)

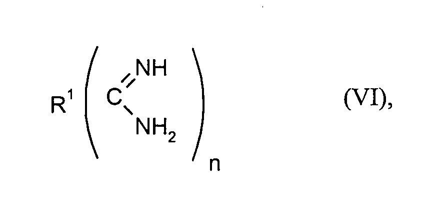

in der
n und R¹ die oben genannten Bedeutungen besitzen
aus den entsprechenden Amidinium-Alkylsulfaten der allgemeinen Formel (V) durch Basenzusatz in an sich bekannter Weise erhalten werden (siehe beispielsweise Acta Chem. Scand., B. 35, 1981, 605).

Weiterhin können die Amidine der allgemeinen Formel (VI) in an sich bekannter Weise in die im Allgemeinen beständigeren Amidin-Kohlendioxid Addukte überführt werden (siehe zum Beispiel Acta Cryst. 1984, C40, 297).

Sowohl die Imidsäureester-Alkylsulfate der allgemeinen Formel (IV) als auch die Amidinium-Alkylsulfate der allgemeinen Formel (V) sind ebenfalls Bestandteil der Erfindung.

Die genannten Verbindungen können zumindest teilweise in tautomeren Formen vorliegen, die vom Umfang der Erfindung ebenfalls umfasst sind. Im Folgenden werden die genannten Reste definiert.

**Alkyl** steht im Rahmen von R¹ und R² beispielsweise für einen geradkettigen oder verzweigten, cyclischen oder acyclischen Alkylrest der weiterhin durch C₁-C₄-Alkoxy substituiert sein kann. C₁-C₄-Alkoxyreste sind Methoxy, Ethoxy, n-Propoxy, iso-Propoxy, n-Butoxy, sek.-Butoxy und tert.-Butoxy.

Die genannten Alkylreste können beispielsweise sein: für C₁-C₄ Methyl, Ethyl, n-Propyl, iso-Propyl, n-Butyl, iso-Butyl oder tert.-Butyl, für C₁-C₆ zusätzlich n-Pentyl, Cyclopentyl, n-Hexyl und für C₁-C₁₂ zusätzlich Cyclohexyl, n-Octyl, iso-Octyl, n-Decyl und n-Dodecyl.

Weiterhin seien beispielsweise genannt Methoxymethyl, Ethoxymethyl, 2-Ethoxyethyl, 2-(2-Ethoxyethoxy)-ethyl, (2-Ethoxyethoxy)-methyl und (2-Methoxyethoxy)-methyl.

Im Rahmen von R³ steht Alkyl beispielsweise für einen geradkettigen oder verzweigten, cyclischen oder acyclischen Alkylrest wie beispielsweise Methyl, Ethyl, n-Propyl, iso-Propyl, n-Butyl, iso-Butyl n-Pentyl, n-Hexyl und Cyclohexyl.

**Alkylen** steht im Rahmen der Erfindung beispielsweise für einen geradkettigen oder verzweigten, cyclischen oder acyclischen Alkylenrest wie beispielsweise Methylen, 1,1-Ethylen, 1,2-Ethylen, 1,3-Propylen, 2,3-Propylen, 2,2-Propylen, 1,4-Butylen, 2,3-Butylen, 1,2-Cyclopentylen und 1,2-Cyclohexylen.

**Alkenyl** steht im Rahmen der Erfindung beispielsweise für einen geradkettigen oder verzweigten Alkenylrest.

Beispielsweise sind dies Vinyl, Allyl, 1-Methylvinyl und 2-Methylvinyl.

**Alkenylen** steht im Rahmen der Erfindung beispielsweise für einen geradkettigen oder verzweigten Alkenylenrest.

Beispielsweise sind dies 1,2-Ethenylen, 1,1-Ethenylen, 1,2-Propenylen, 2,3-Butenylen, 2-Methyl-1,1-ethenylen und 2,2-Dimethyl-1,1-ethenylen.

**Halogenalkyl** steht im Rahmen der Erfindung beispielsweise für einen geradkettigen oder verzweigten, cyclischen oder acyclischen Alkylrest, in dem eines, mehrere oder alle Wasserstoffatome durch Halogenatome substituiert sind die unabhängig voneinander ausgewählt sind aus der Gruppe Chlor und Fluor.

Beispielsweise sind dies Fluormethyl, Chlormethyl, Dichlormethyl, Difluormethyl, Chlorfluormethyl, Dichlorfluormethyl, Chlordifluormethyl, Trifluormethyl, Trichlormethyl, 2,2,2-Trichlorethyl, Pentafluorethyl, Heptafluor-n-propyl und Nonafluor-nbutyl.

**Arylalkyl** steht im Rahmen der Erfindung beispielsweise für einen geradkettigen oder verzweigten Alkylrest der durch wenigstens einen Arylrest substituiert ist. Der oder die Arylreste können ihrerseits weiterhin substituiert sein. Als Arylrest bevorzugt ist Phenyl.

Arylalkylreste können beispielsweise sein: Benzyl, 1-Methylbenzyl, o-, m-, p-Methylbenzyl sowie o-, m-, p-Methoxybenzyl.

Beispiele für Substituenten der allgemeinen Formel (II) sind:
Cyanomethyl, Cyanoethyl, Methoxycarbonylmethyl, Methoxycarbonylethyl, Ethoxycarbonyl-methyl, Ethoxycarbonylethyl und Acetylmethyl.

Bevorzugt werden für das erfindungsgemäße Verfahren als Carbonsäureamide solche der allgemeinen Formel (I) eingesetzt, wobei n = 1 ist und R¹ für C₁-C₆-Alkyl, C₁-C₆-Halogenalkyl oder Benzyl steht.

Besonders bevorzugt werden für das erfindungsgemäße Verfahren Carbonsäureamide der allgemeinen Formel (I) eingesetzt, wobei n = 1 ist und R¹ für Methyl, Ethyl oder 1-Methylethyl steht.

Ganz besonders bevorzugt wird für das erfindungsgemäße Verfahren Essigsäureamid (Acetamid) eingesetzt.

Bevorzugt wird für das erfindungsgemäße Verfahren nur ein Schwefelsäurediester der allgemeinen Formel (III) eingesetzt.

Bevorzugte Schwefelsäurediester der allgemeinen Formel (III) sind solche in denen beide Reste R³ identisch sind und für Methyl, Ethyl, n-Propyl oder n-Butyl stehen.

Besonders bevorzugt ist Schwefelsäuredimethylester (Dimethylsulfat).

Gegebenenfalls kann Schritt a) in Gegenwart von Lösungsmittel durchgeführt werden.

Geeignete Lösungsmittel für Schritt a) sind beispielsweise aliphatische oder aromatische Kohlenwasserstoffe wie n-Hexan, die isomeren Hexane oder Mischungen davon, Cyclohexan, Toluol, o-, m-, p-Xylol, halogenierte Kohlenwasserstoffe wie zum Beispiel Chloroform, Dichlorethan oder Chlorbenzol, Ether wie Tetrahydrofuran, Dioxan, Dimethoxyethan (Glyme), Diethoxyethan, Diglyme oder Methyl-tert.butylether oder Mischungen solcher Lösungsmittel.

Bevorzugt ist die Durchführung des Schrittes a) ohne Lösungsmittel.

Die Reaktionstemperatur für Schritt a) kann beispielweise 20 bis 130°C, bevorzugt 50 bis 100°C betragen.

Der Druck ist im Allgemeinen nicht kritisch und kann beispielsweise 0,1 bis 20 bar betragen, bevorzugt sind 0,8 bis 1,2 bar.

Das molare Verhältnis von Carbonsäureamid-Gruppen zu Schwefelsäurediester der allgemeinen Formel (III) kann beispielsweise 0,4 bis 3 betragen, 0,9 bis 1,5 ist bevorzugt, 1,0 bis 1,1 besonders bevorzugt. Der Einsatz größerer Mengen von Schwefelsäureester ist möglich aber unwirtschaftlich.

Man kann Schritt a) vorzugsweise so durchführen, dass man den Schwefelsäurediester gegebenenfalls in Lösungsmittel vorlegt und das Carbonsäureamid gegebenenfalls in Lösungsmittel gelöst zugibt.

Besonders bevorzugt ist die Zugabe des Carbonsäureamids zum Schwefelsäurediester jeweils in Substanz.

Die auf diese Weise erhaltenen Imidsäureester-Alkylsulfate der allgemeinen Formel (IV) können aufgrund ihrer überraschenden Stabilität sowohl isoliert und gelagert oder direkt weiter umgesetzt werden. Bevorzugt ist die direkte Weiterumsetzung.

Schritt b) kann gegebenenfalls in Gegenwart von Lösungsmitteln durchgeführt werden. Geeignete Lösungsmittel für Schritt b) sind beispielsweise aliphatische oder aromatische Kohlenwasserstoffe wie n-Hexan, die isomeren Hexane oder Mischungen davon, Cyclohexan, Toluol, o-, m-, p-Xylol, halogenierte Kohlenwasserstoffe wie zum Beispiel Chloroform, Dichlorethan oder Chlorbenzol, Ether wie Tetrahydrofuran, Dioxan, Dimethoxyethan (Glyme), Diethoxyethan, Diglyme oder Methyl-tert.butylether, Alkohole wie Methanol, Ethanol, iso-Propanol, n-Butanol, iso-Butanol oder tert.-Butanol oder Mischungen solcher Lösungsmittel.

Bevorzugt wird Schritt b) ohne Lösungsmittel oder in Gegenwart von Methanol, Ethanol oder iso-Propanol oder Mischungen dieser Alkohole durchgeführt.

Besonders bevorzugt wird Schritt b) in Gegenwart von Methanol durchgeführt.

Das molare Verhältnis von Ammoniak zu Imidsäureester-Alkylsulfat-Gruppen kann beispielsweise 0,4 bis 3 betragen, bevorzugt zwischen 0,9 und 1,5 besonders bevorzugt zwischen 1.0 und 1.2. Eine größere Menge von Ammoniak ist möglich und kann in verflüssigter Form zum Beispiel selbst als Solvens dienen.

Die Reaktionstemperatur kann beispielsweise zwischen -40 und 100°C betragen, bevorzugt zwischen 10 und 50°C.

Der Druck der Reaktion kann beispielsweise 0,5 bis 100 bar, bevorzugt zwischen 0,8 und 2 bar liegen, besonders bevorzugt sind 0,8 bis 1,2 bar. Bei Durchführung des erfindungsgemäßen Verfahrens ohne Lösungsmittel ist der Druck so zu wählen, dass der eingesetzte Ammoniak bei der gewählten Reaktionstemperatur zumindest teilweise flüssig ist.

Vorzugsweise werden zur Aufarbeitung gegebenenfalls eingesetztes Lösungsmittel, der bei der Reaktion freigesetzte Alkohol und gegebenenfalls überschüssiger Ammoniak entfernt. Dies kann beispielsweise durch Destillation zum Beispiel unter vermindertem Druck erfolgen.

In einer bevorzugten Ausführungsform legt man eine alkoholische Ammoniaklösung vor und dosiert das Imidsäureester-Alkylsulfat der allgemeinen Formel (IV) gegebenenfalls in Lösungsmittel gelöst zu.

Flüssige Imidsäureester-Alkylsulfate der allgemeinen Formel (IV) werden bevorzugt ohne Lösungsmittel zudosiert.

Die auf diese Weise erhaltenen Amidinium-Alkylsulfate der allgemeinen Formel (V) können entweder gelagert oder direkt weiter umgesetzt werden.

Zur weiteren Umsetzung kann man aus den Amidinium-Alkylsulfaten der allgemeinen Formel (V) beispielsweise die freien Amidine durch Basenzusatz in an sich bekannter Weise erhalten. Geignete Basen sind beispielsweise Alkalimetall-alkoholate oder ―hydride. Bevorzugt sind Natrium- und Kalium-methanolat, -ethanolat, isopropanolat, und -tert.-butanolat. Besonders bevorzugt ist Natriummethanolat.

Gegebenenfalls können die freien Amidine in an sich bekannter Weise in ein Kohlendioxid-Addukt überführt werden, wobei bevorzugt die Herstellung durch Einleiten von Kohlendioxid in eine Lösung des freien Amidins erfolgt.

Die erfindungsgemäßen Amidinium-Alkylsulfate eignen sich insbesondere für Verfahren zur Herstellung stickstoffhaltiger Heterocyclen und von Verbindungen, die stickstoffhaltige Heterocyclen enthalten. Beispiele für solche stickstoffhaltige Heterocyclen sind Imidazole oder Pyrimidine. Verbindungen, die stickstoffhaltige Heterocyclen enthalten, sind beispielsweise Vitamin B1, sowie in Arzneimitteln verwendete Substanzen wie zum Beispiel Conivaptan und Eprosartan sowie die Derivate dieser Verbindungen.

Der Vorteil des erfindungsgemäßen Verfahrens ist, dass die Amidinium-Alkylsulfate ausgehend von den im Vergleich zu den entsprechenden Nitrilen wenig toxischen Carbonsäureamiden in außerordentlich hohen Ausbeuten hergestellt werden können. Darüberhinaus zeigen die erfindungsgemäßen Imidsäureester-Alkylsulfate und Amidinium-Alkylsulfate überraschenderweise eine hohe Stabilität und Löslichkeit, was deren Verarbeitung im Vergleich zu den bekannten Verfahren erheblich erleichtert. Darüberhinaus sind die genannten Verbindungen zumindest teilweise flüssig, was die Verarbeitung ebenfalls erheblich vereinfacht. Die leichte Handhabung macht auch deren Einsatz in einem Verfahren zur Herstellung von Verbindungen, die stickstoffhaltige Heterocyclen enthalten, besonders vorteilhaft.

### Beispiele

### Beispiel 1

a) In einem 1-1-Einhalskolben wurden 378 g (3 mol) Dimethylsulfat vorgelegt und unter Überleiten von trockenem Stickstoff auf 65° C aufgeheizt. Innerhalb von 30 min wurden anschließend 177 g (3 mol) Acetamid portionsweise zudosiert. Nach Zugabe von einem Drittel der Gesamtmenge wurde leicht gekühlt, um die Temperatur auf 65° C zu halten. Nach Ende der Zugabe wurde noch 2 h bei 70° C gerührt, dann ließ man abkühlen.
b) In einem zweiten 1-1-Einhalskolben wurden in 300 ml trockenes Methanol 60g Ammoniak eingeleitet. Anschließend ließ man innerhalb 30 min bei Raumtemperatur das Produkt aus a) unter schwacher Kühlung zutropfen. Es wurde noch 1 h bei Raumtemperatur nachgerührt, und anschließend bei etwa 50°C und etwa 10 mbar die flüchtigen Bestandteile abgezogen. Man erhielt 504,2 g flüssiges Rohprodukt, das laut H-NMR zu 96,4% aus Acetamidinium-Methylsulfat bestand. Dies entspricht einer Ausbeute von 95,3 % d. Th. über beide Stufen.
¹H-NMR Imidessigsäuremethylester-Methylsulfat (CDCl₃): 2,49 (s, 3H, CH₃-C); 3,73 (s, 3H, MeOSO₃⁻); 4,23 (s, 3H, OMe); 10,35, 11,0 (jeweils s(breit), 2H, NH₂) ¹H-NMR Acetamidinium-Methylsulfat (CDCl₃): 2,30 (s, 3H, CH₃-C); 3,68 (s, 3H, MeOSO₃⁻); 8,03, 8,22 (jeweils s(breit), 3H, C=NH(NH₂)).

### Beispiel 2

73,1g Propionsäureamid wurden analog zu Beispiel 1, jedoch mit Hilfe einer Dosierschnecke mit 126g Dimethylsulfat umgesetzt. Der Imidsäureester wurde dann in eine Lösung von 20g Ammoniak in 100 ml Methanol getropft. Nach 1 h Nachrührzeit zog man bei 40 ― 45° C und 5 mbar die flüchtigen Bestandteile ab. Es wurden 182,4 g Rohprodukt erhalten, das laut H-NMR zu 96,0 % aus Propansäureamidinium-Methylsulfat bestand, entsprechend 95,1 % d. Th..

### Beispiel 3

Analog zu Beispiel 2 wurde aus iso-Butansäureamid iso-Buttersäureamidinium-Methylsulfat in 93,7% d. Th. erhalten.

## Patentansprüche

1. Verfahren zur Herstellung von Amidinium-Salzen, **dadurch gekennzeichnet, dass** Carbonsäureamide der allgemeinen Formel (I) 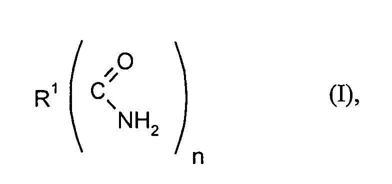 in der
n = 1 oder 2 und abhängig von n
für n = 1
R¹ für C₁-C₁₂-Alkyl, C₂-C₈-Alkenyl, C₁-C₁₂-Halogenalkyl, oder C₇-C₁₂-Arylalkyl oder für Substituenten der allgemeinen Formel (II) steht,
A-B-D (II)
in der unabhängig voneinander
A für einen C₁-C₄-Alkylenrest und
B für eine Carbonylgruppe und
D für R² oder OR² steht,
wobei R² C₁-C₆-Alkyl bedeutet
oder
B und D zusammen für eine Cyanogruppe stehen,
und für n = 2
R¹ für C₁-C₆-Alkylen oder C₂-C₆-Alkenylen steht,
a) mit mindestens einem Schwefelsäurediester der allgemeinen Formel (III) 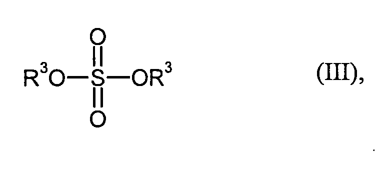 in der die Reste R³ jeweils unabhängig voneinander für C₁-C₆-Alkyl stehen,
zu Imidsäureester-Alkylsulfaten der allgemeinen Formel (IV) 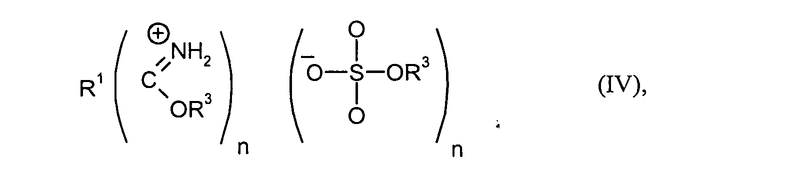 in der
n, R¹ und jeweils unabhängig voneinander die Reste R³ die oben genannte Bedeutung besitzen., umgesetzt werden und
b) diese dann mit Ammoniak zu den Amidinium-Alkylsulfaten der allgemeinen Formel (V) umgesetzt werden, 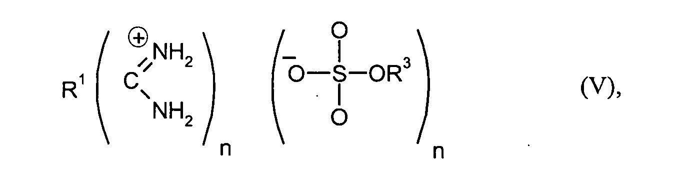 in der
n, R¹ und jeweils unabhängig voneinander die Reste R³ die oben genannte Bedeutung besitzen.

2. Verfahren nach Anspruch 1, **dadurch gekennzeichnet, dass** Schritt b) in Gegenwart von Lösungsmittel durchgeführt wird.

3. Verfahren nach einem oder mehreren der Ansprüche 1 bis 2, **dadurch gekennzeichnet, dass** Schritt a) bei 30 bis 130°C durchgeführt wird.

4. Verfahren nach einem oder mehreren der Ansprüche 1 bis 3, **dadurch gekennzeichnet, dass** Schritt b) bei einer Temperatur von ―40 bis 100°C durchgeführt wird.

5. Verfahren nach einem oder mehreren der Ansprüche 1 bis 4, **dadurch gekennzeichnet, dass** als Schwefelsäurediester Dimethylsulfat verwendet wird.

6. Imidsäureester-Alkylsulfate der allgemeinen Formel (IV) 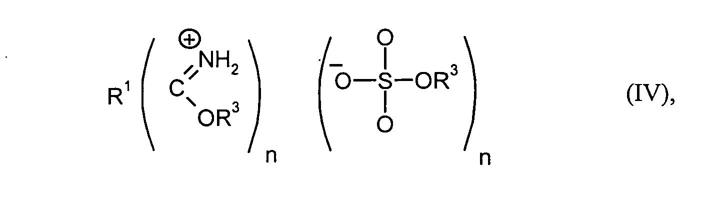 in der
n für 1 oder 2 und abhängig von n
für n = 1
R¹ für C₁-C₁₂-Alkyl, C₂-C₈-Alkenyl, C₁-C₁₂-Halogenalkyl, oder C₇-C₁₂-Arylalkyl oder für Substituenten der allgemeinen Formel (II)
A-B-D (II)
in der unabhängig voneinander
A für einen C₁-C₄-Alkylenrest und
B für eine Carbonylgruppe und
D für R² oder OR² steht
wobei R² C₁-C₆-Alkyl bedeutet
oder B und D zusammen für eine Cyanogruppe stehen,
und für n = 2
R¹ für C₁-C₆-Alkylen oder C₂-C₆-Alkenylen steht
und die Reste R³ jeweils unabhängig voneinander für einen C₁-C₆-Alkylrest stehen.

7. Imidessigsäuremethylester-Methylsulfat.

8. Amidinium-Alkylsulfate der allgemeinen Formel (V) 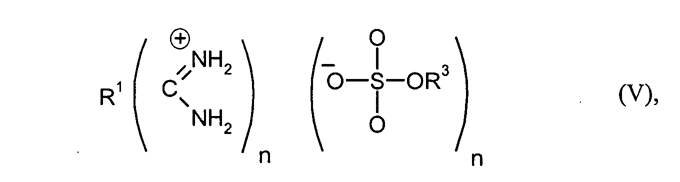 in der
n für 1 oder 2 und abhängig von n
für n = 1
R¹ für C₁-C₁₂-Alkyl, C₂-C₈-Alkenyl, C₁-C₁₂-Halogenalkyl, oder C₇-C₁₂-Arylalkyl oder für Substituenten der allgemeinen Formel (II)
A-B-D (II)
in der unabhängig voneinander
A für einen C₁-C₄-Alkylenrest und
B für eine Carbonylgruppe und
D für R² oder OR² steht
wobei R² C₁-C₆-Alkyl bedeutet
oder B und D zusammen für eine Cyanogruppe stehen,
und für n = 2
R¹ für C₁-C₆-Alkylen oder C₂-C₆-Alkenylen steht
und die Reste R³ jeweils unabhängig voneinander für einen C₁-C₆-Alkylrest stehen.

9. Acetamidinium-Methylsulfat.

10. Verfahren zur Herstellung von Amidinen, **dadurch gekennzeichnet, dass** Amidinium-Alkylsulfate gemäß Ansprüchen 8 oder 9 mit Base umgesetzt werden.

11. Verfahren zur Herstellung von stickstoffhaltigen Heterocyclen, **dadurch gekennzeichnet, dass** man Imidsäureester-Alkylsulfate gemäß Anspruch 6 oder Amidinium-Alkylsulfate gemäß Anspruch 8 oder Amidine gemäß Anspruch 10 oder deren Kohlendioxid Addukte einsetzt.

12. Verfahren zur Herstellung von Amidin-Kohlendioxid-Addukten, **dadurch gekennzeichnet, dass** man Imidsäureester-Alkylsulfate gemäß Anspruch 6 oder Amidinium-Alkylsulfate gemäß Anspruch 8 einsetzt.

13. Verwendung von Imidsäureester-Alkylsulfaten gemäß Anspruch 6 in einem Verfahren zur Herstellung von Arzneimitteln.

14. Verwendung von Amidinium-Alkylsulfaten gemäß Anspruch 8 in einem Verfahren zur Herstellung von Arzneimitteln.

15. Verwendung von Imidsäureester-Alkylsulfaten gemäß Anspruch 6 in einem Verfahren zur Herstellung von Vitamin B1.

16. Verwendung von Amidinium-Alkylsulfaten gemäß Anspruch 8 in einem Verfahren zur Herstellung von Vitamin B1.

17. Verwendung von Amidinen gemäß Anspruch 10 oder deren Kohlendioxid-Addukte in einem Verfahren zur Herstellung von Arzneimitteln.

18. Verfahren zur Herstellung von Arzneimitteln, **dadurch gekennzeichnet, dass** man Imidsäureester-Alkylsulfate gemäß Anspruch 6 einsetzt.

19. Verfahren zur Herstellung von Arzneimitteln, **dadurch gekennzeichnet, dass** man Amidinium-Alkylsulfate gemäß Anspruch 8 oder Amidine gemäß Anspruch 10 oder deren Kohlendioxid-Addukte einsetzt.
